# EUROPEAN PATENT SPECIFICATION

(11) **EP 3 858 263 B1**
(45) Date of publication and mention of the grant of the patent: **20.12.2023**
(21) Application number: 19870690.5
(22) Date of filing: 08.10.2019
(51) Int. Cl.: A61B 17/12

(54) **EMBOLISM DEVICE AND SPRING COILS THEREOF**
EMBOLIEVORRICHTUNG UND FEDERWINDUNGEN DAFÜR
DISPOSITIF D'EMBOLISATION ET SES SPIRES DE RESSORT

(30) Priority: 09.10.2018 CN 201811170237
(43) Date of publication of application: 04.08.2021
(73) Proprietor: MicroPort NeuroTech (Shanghai) Co., Ltd., Shanghai 201318 (CN)
(72) Inventor: GUO, Yuanyi, Shanghai 201318 (CN); PENG, Yunfei, Shanghai 201318 (CN); YU, Peng, Shanghai 201318 (CN); ZHA, Xu, Shanghai 201318 (CN); CHEN, Bing, Shanghai 201318 (CN); WANG, Yiqun Bruce, Shanghai 201318 (CN)
(74) Representative: Marks & Clerk LLP
(86) International application number: PCT/CN2019/110019
(87) International publication number: WO 2020/073902

(56) References cited:
- WO-A1-2017/105479
- US-A1- 2017 105 738
- US-A1- 2018 104 040

## Description

### TECHNICAL FIELD

The present invention relates to the field of medical instruments and, in particular, an embolization device and an embolization coil thereof for use in the treatment of intracranial aneurysms.

### BACKGROUND

Brain aneurysms, also known as intracranial aneurysms, are a severe threat to our health. With the development of imaging technology and biomaterials for intravascular use, intravascular intervention has replaced surgical clipping as the first choice therapy for intracranial aneurysms thanks to its lower risk and less trauma.

WO2017105479A1 describes how vascular issues may be addressed with systems, devices and methods for delivering implants with accurate and ready detachability, among other features, for addressing, for example, acute stroke issues with due alacrity

Presently, the treatment of an aneurysm is usually accomplished with a coil. During embolization of the aneurysm, a corresponding shape is formed in the aneurysm by a predetermined shape of the coil, thereby achieving a desired embolization effect. For such treatment, the initial packing density of the coil is one important factor that determines long-term stability of the embolization effect, and a greater coil packing density can result in better clinical treatment outcomes.

Existing coils include three- and two-dimensional structures. In most cases, a desired coil shape is made up of a combination of both particular three- and two-dimensionally structures. The three-dimensionally structures are designed to build a stable frame in the lumen of an aneurysm and provide support to the neck of the aneurysm and the two-dimensionally ones to fill open spaces in the aneurysm to achieve a desired packing density. However, the three-dimensional structures of existing coils have a significant drawback that it is difficult for them to achieve both stable basket formation and compliant packing. For example, their constituent elements are simple figure-eight shaped, O-shaped or Ω-shaped coils, which are difficult to compress and poorly compliant, making the coils unsuitable for the embolization of different aneurysms of various shapes and sizes.

### SUMMARY OF THE INVENTION

The invention is defined in independent claim 1. Certain optional features of the invention are defined in the dependent claims. In view of this, the present invention provides an embolization device and an embolization coil thereof with both sufficient stability and satisfactory compliance, which can adapt to aneurysms of different shapes and sizes and allow stable, compliant, dense embolization thereof.

According to one aspect of the present invention, there is proposed a coil formed by joining together at least four structural elements arranged in different planes. The at least four structural elements include at least two C-shaped elements, at least one O-shaped element and at least one Ω-shaped element, and the at least two C-shaped elements are arranged in two adjacent planes and are sequentially joined together to form an S-shaped structure. In the coil, the Ω-shaped element has an opening smaller than an opening of the C-shaped element.

Additionally, the two adjacent planes where the S-shaped structure is arranged may form an angle of 60°-120°.

Additionally, the angle of the two adjacent planes where the S-shaped structure is arranged may be 80°-100°.

Additionally, the angle formed by the two adjacent planes where the S-shaped structure is arranged may be 90°.

Additionally, the Ω-shaped element may have an arc length greater than or equal to 75% and smaller than 100% of a complete circumference, and the C-shaped element may have an arc length greater than or equal to 50% and smaller than 75% of a complete circumference.

Additionally, in the coil, the total number of the structural elements may be six, and the number of the C-shaped elements may be two.

Additionally, the six structural elements may make up a hexahedron having three axes on each, and one Ω-shaped element is arranged on each of three axes of the hexahedron.

Additionally, in the coil, the coil may be formed by sequentially joining together an O-shaped element, a Ω-shaped element, one of the C-shaped elements, the other one of the C-shaped elements, another Ω-shaped element and a further Ω-shaped element, which are respectively arranged in different planes.

Additionally, in the coil, the total number of the structural elements may be eight, and the number of the C-shaped elements may be six.

Additionally, in the coil, the coil may be formed by sequentially joining together an O-shaped element, one of the C-shaped elements, another one of the C-shaped elements, a further one of the C-shaped elements, a further one of the C-shaped elements,
a further one of the C-shaped elements, the remaining one of the C-shaped elements and a Ω-shaped element, which are respectively arranged in different planes.

Additionally, the coil may be formed by winding a tubular body.

Additionally, in the coil, the tubular body may be made of a metal, alloy or polymer filament and is wound into a spiral.

According to another aspect of the present invention, there is proposed an embolization device comprising at least one coil as defined above.

Additionally, the embolization device may include a plurality of coils, wherein the plurality of coils are joined together side-by-side and end-to-end, and wherein in any adjacent two coils, one coil is swiveled about an axis of the embolization device with respect to the other coil.

Additionally, in the embolization device, in any adjacent two coils, one coil may be swiveled about the axis of the embolization device with respect to the other coil by an angle of 0°-90°.

Additionally, in the embolization device, the structural element arranged at a furthermost end of the embolization device is the O-shaped element.

In the proposed embolization device and coil thereof, the coil is formed by joining together at least four structural elements arranged in different planes, which include at least two C-shaped elements and at least one O-shaped or Ω-shaped element. The at least two C-shaped elements are arranged in two adjacent planes and sequentially joined together to form an S-shaped structure. The three-dimensional S-shaped structure is highly deflectable and compressive, making the coil easy to change its shape to adapt to different aneurysm shapes. Moreover, the Ω- and O-shaped elements are highly resistant to compression and can provide strong support and ensure sufficient stability of the coil.

With the advantages of both types of structural elements, the coil has both good stability and good compliance. Therefore, it can adapt to aneurysms of different shapes and sizes, and enables stable, compliant, dense embolization.

Further, the two adjacent planes where the S-shaped structure is arranged preferably form an angle of 60°-120°, with 90° more preferred. This can result in an additional increase in spatial deflectability of the S-shaped structure and hence in compliance of the coil.

Furthermore, the embolization device may include a plurality of coils, for example, 2-10 coils, in which any adjacent two can be swiveled with respect to each other about the axis of the embolization device. This imparts higher stability to the embolization device, thereby resulting in an even better aneurysm embolization effect.

### BRIEF DESCRIPTION OF THE DRAWINGS

The accompanying drawings are provided for a better understanding of the effect.

### BRIEF DESCRIPTION OF THE DRAWINGS

The accompanying drawings are provided for a better understanding of the present invention and do not limit it in any way. In these figures:
Fig. 1 is a structural schematic of a coil according to a first embodiment of the present invention, which is in the shape of a hexahedron;
Fig. 2 schematically illustrates a Ω-shaped element in the coil of Fig. 1;
Fig. 3 schematically illustrates an S-shaped structure made up of two C-shaped elements in the coil of Fig. 1;
Fig. 4 is a simplified schematic of a coil according to a second embodiment of the present invention, which is in the shape of a pentahedron;
Fig. 5 is a simplified schematic of an embolization device according to a fourth embodiment of the present invention, which includes two hexahedron-shaped coils not swiveled to each other;
Fig. 6 is a diagram of the embolization device of Fig. 5, in which the two coils are swiveled to each other;
Fig. 7 is a simplified schematic of the embolization device according to the fourth embodiment of the present invention, which includes two pentahedron-shaped coils swiveled to each other; and
Fig. 8 is a simplified schematic of the embolization device according to the fourth embodiment of the present invention, which includes two octahedron-shaped coils not swiveled to each other.

In these figures,
10, 20, 30: a coil; 11: a Ω-shaped element; 12: a C-shaped element; and 13: an O-shaped element.

### DETAILED DESCRIPTION

The present invention will be described in greater detail below by way of particular embodiments with reference to the accompanying drawings. Advantages and features of the present invention will be more apparent from the following detailed description. Note that the figures are provided in a very simplified form not necessarily drawn to exact scale, and they are only intended to facilitate convenience and clarity in explaining the disclosed embodiments.

As used herein and in the appended claims, the singular forms "a", "an", and "the" include plural references unless the context clearly dictates otherwise. As used herein and in the appended claims, the term "or" is generally employed in its sense including "and/or" unless the content clearly dictates otherwise. The term "C-shaped" is intended to refer to semicircular or similar open loops of shapes including, but not limited to, circular arcs, elliptical arcs, those consisting of circular arcs with different curvatures, and those partially linear and partially arcuate. The term "Ω-shaped" is intended to refer to an open loop with smaller openings than "C-shaped" ones. Likewise, Ω-shaped open loops may also be of shapes including, but not limited to, circular arcs. The term "O-shaped" is intended to refer to closed loops of shapes including, but not limited to, circles, ellipses or irregular shapes. Those skilled in the art will recognize that the term "closed loops" is intended to refer to loops without a seam throughout the total length rather than those formed by bringing opposing ends together.

The following description sets forth numerous specific details in order to provide a more thorough understanding of the present invention. However, it will be apparent to those skilled in the art that the present invention can be practiced without one or more of these specific details. In other instances, well-known technical features have not been described in order to avoid unnecessary obscuring of the present invention.

Before describing the present invention in detail, the primary principles and concept of the present invention will be briefed first. A three-dimensional coil shape can be formed by joining together two-dimensional Ω-shaped structures and three-dimensional S-shaped structures arranged in different planes. The Ω-shaped structures are structurally stable, allowing the coil to maintain good structural stability. At the same time, the three-dimensional S-shaped structures have good deflectability and compressibility, which impart high compliance to the coil. With this construction, the coil can achieve both stable basket formation and compliant packing and can adapt to various aneurysms of different shapes and sizes with a dense packing effect.

According to embodiments of the present invention, a coil is formed by joining together at least four structural elements arranged in different planes. The at least four structural elements include at least two C-shaped elements and at least one O-shaped or Ω-shaped element. The at least two C-shaped elements are arranged in two adjacent ones of the planes and sequentially joined to form an S-shaped structure. As used herein, the term "O-shaped element" is meant to refer to a closed loop structure, which may be composed of multiple Ω- or C-shaped elements arranged in the same plane of the coil. For example, an O-shaped element may be made up of 1.5 Ω-shaped elements or 2 C-shaped elements. Both the Ω- and C-shaped elements are open loop structures, and each C-shaped element has an opening greater than the opening of the Ω- -shaped elements, and hence the C-shaped element has a higher compressibility than any Ω-shaped one.

According to embodiments of the present invention, the coil is formed by winding a tubular body.

### <Embodiment 1>

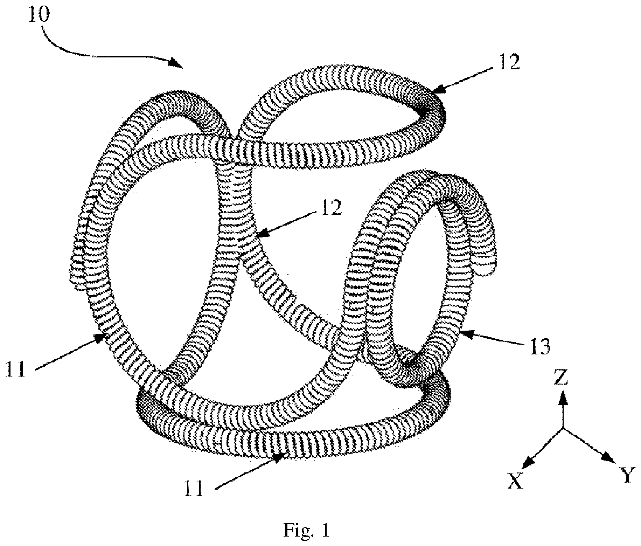

As shown in Figs. 1 to 3, a coil 10 according to this embodiment is formed by joining together structural elements, which are of three types and arranged in six planes. The tructural elements include O-, Q- and C-shaped elements 13, 11, 12.There is one O-shaped element 13 and al least two C-shaped elements 12, and thetotal number of the O-, Q- and C-shaped elements 13, 11, 12 is six. The six structural elements are arranged in the respective six planes so that the coil 10 generally appears as a hexahedron. Examples of the hexahedron are not limited to regular hexahedrons, parallel hexahedrons and irregular hexahedrons. In other embodiments, the six structural elements may also be arranged in respective six planes of a polyhedron having at least seven planes, such as an octahedron.

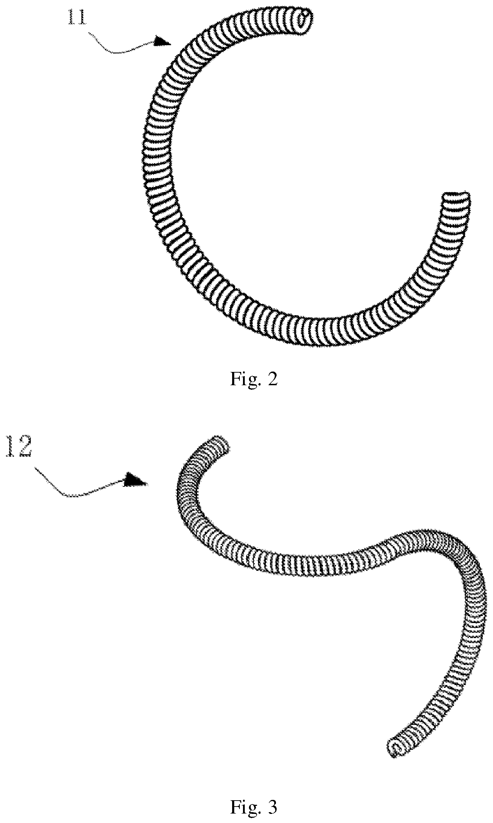

The O-, Ω- and C-shaped elements 13, 11, 12 may be arranged in many ways, as long as they satisfy several requirements including: preferred arrangement of the O-shaped element 13 at the furthermost end of the coil 10 (i.e., the end farthest away from a pusher rod), i.e., the O-shaped element 13 being preferred to be a leading structural element in the formation of the tubular body, which can effectively ensure good stability of the resulting coil; and the formation of at least one three-dimensional S-shaped structure by two C-shaped elements 12 arranged in adjacent planes, which can impart increased compliance to the coil 10. As shown in Fig. 3, the two C-shaped elements 12 may be joined at an angle (preferably, tangentially to each other). The three-dimensional S-shaped structure possesses good deformation properties including high deflectability and high compressibility, which are conducive to increased compliance of the coil 10 and make it easier to adapt to the embolization requirements of different aneurysms. At the same time, the high compression resistance of the Ω- and O-shaped elements 11, 13 can ensure sufficient stability of the coil during its stay in the lumen of the aneurysm.

It should be noted that each Ω-shaped element 11 has a smaller opening than the C-shaped elements 12. In other words, each Ω-shaped element 11 is an open loop with an opening that is smaller than those of -4 the C-shaped elements 12. Preferably, each Ω-shaped element 11 has an arc length greater than or equal to 75% and smaller than 100% of a length of the corresponding complete circumference. Therefore, it is hard to compress, stable and conducive to increased stability of the coil. Each C-shaped element 12 has a greater opening and is preferred to have an arc length greater than or equal to 50% and smaller than 75% of a length of the corresponding complete circumference. Therefore, it is easy to compress and compliant. Thus, incorporating both these types of structural elements allows the coil to have both good stability and good compliance.

In the embodiment illustrated in Fig. 1, there is one O-shaped element 13, two C-shaped elements 12 and three Ω-shaped elements 11 in the coil 10, which are sequentially joined together in the following sequence: the O-shaped element 13, one of the Ω-shaped elements 11, one of the C-shaped elements 12, the other one of the C-shaped elements 12, another one of the Ω-shaped elements 11 and the remaining one of the Ω-shaped elements 11. One of the C-shaped elements 12 may be arranged at a top side of the hexahedron, and the other C-shaped element 12 may be arranged at a rear side of the hexahedron, i.e., the furthermost side as viewed in Fig. 1. This arrangement is advantageous in that the three Ω-shaped elements 11 are arranged on respective axes of the hexahedron, with one O-shaped element being arranged on one of the axes. This can ensure good stability of the coil 10, in addition to good overall compliance thereof assured by the three-dimensional S-shaped structure.

Preferably, the two adjacent planes where the S-shaped structure is arranged form an angle of 60°-120°. In some embodiments, the angle of the two adjacent planes where the S-shaped structure is arranged may be 80°-100°. In some embodiments, the angle of the two adjacent planes where the S-shaped structure is arranged may be 80°, 90° or 100°. In addition, in the case of the angle of the two adjacent planes where the S-shaped structure is arranged being 90°, the S-shaped structure will have the highest deflectability in the three-dimensional space, resulting in better compliance of the coil 10.

Apart from the above-described construction, in alternative embodiments, the coil 10 may be constructed by joining together one O-shaped element 13, three C-shaped elements 12 and two Ω-shaped elements 11 sequentially in the following order: the O-shaped element 13, one of the Ω-shaped elements 11, one of the C-shaped elements 12, another one of the C-shaped elements 12, the other one of the Ω-shaped elements 11 and the remaining one of the C-shaped elements 12. In yet alternative embodiments, the coil 10 may be constructed by joining together one O-shaped element 13, four C-shaped elements 12 and one Ω-shaped element 11 sequentially in the following order: the O-shaped element 13, the Ω-shaped element 11, one of the C-shaped elements 12, another one of the C-shaped elements 12, a further one of the C-shaped elements 12 and the remaining one of the C-shaped elements 12. In still alternative embodiments, the coil 10 may be constructed by joining together one O-shaped element 13, three C-shaped elements 12 and two Ω-shaped elements 11 sequentially in the following order: the O-shaped element 13, one of the C-shaped elements 12, another one of the C-shaped elements 12, one of the Ω-shaped elements 11, the other one of the Ω-shaped elements 11 and the remaining one of the C-shaped elements 12. It would be appreciated that, in case of an odd number of C-shaped elements 12, e.g., three C-shaped elements 12, since each C-shaped element 12 has a greater opening than those of the Ω-shaped elements 11, the greater the number of the C-shaped elements 12 is, the better the deformability of the coil 10 will be.

In general, the hexahedron may have three axes, e.g., the X, Y and Z axes as shown in Fig. 1. In case of a regular hexahedron, the three axes are orthogonal to one another. In order to achieve both good stability and sufficient compliance, in addition to forming one S-shaped structure by arranging respective C-shaped elements 12 in two adjacent ones of the planes, it is preferred that in each pair of opposing ones of the planes, which are both perpendicular to a corresponding one of the axes, one Ω-shaped element 11 and one C-shaped element 12 are respectively arranged. Specifically, when the embolization device includes only one coil 10, or when the coil 10 is arranged at the furthermost (or closest) end of the embolization device, it is preferred that the leading first element (or the trailing element, in case of the coil 10 is arranged at the closest end) is a closed loop, in order to ensure that the embolization device has sufficient stability at said end. In other instances, the O-shaped element 13 may be replaced with a Ω-shaped element 11, or the O-shaped element 13 may not be the leading element. Additionally, the coil 10 may include more than one O-shaped element 13. The O-shaped element 13 is a spiral structure formed by winding the tubular body of the coil. As used herein, the term "spiral structure" can be interpreted to refer to a structure consisting of multiple Ω- or C-shaped elements 11, 12 of the same or different diameters, such as a structure consisting of 1.5 Ω-shaped elements 11 as shown in Fig. 1. The closed loop can increase stability of the coil.

Further, the hexahedron is preferred to have an angle of 90° between each pair of planes where two corresponding adjacent ones of the elements are arranged. The hexahedron is preferably a regular hexahedron, i.e., a cube. Those skilled in the art may make suitable modifications to the foregoing element arrangements in light of the teachings herein to obtain coils 10 with different element arrangements, which are, however, intended to also fall within the scope of the invention.

### <Embodiment 2>

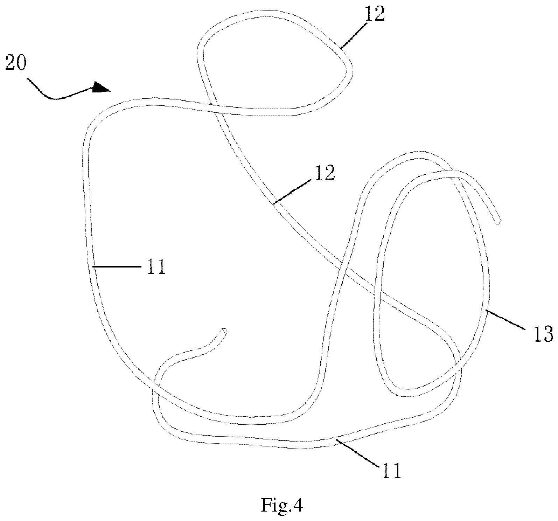

As shown in Fig. 4, a coil 20 according to this embodiment is formed by joining together structural elements, which are of three types and arranged in five planes. Similarly, the structural elements include O-, Ω- and C-shaped elements 13, 11, 12. There is one O-shaped element 13 and at least two C-shaped elements 12, and the total number of the O-, Ω- and C-shaped elements 13, 11, 12 is five. The five structural elements are arranged in the respective five planes to achieve a coil 20 with a pentahedron structure. Herein, the coil 20 appears as a pentahedron. In other embodiments, the five structural elements may also be arranged in respective five planes of a polyhedron having at least six planes, such as a hexahedron.

In the pentahedron, the O-, Ω- and C-shaped elements 13, 11, 12 may also be arranged in many ways, and the arrangement may follow substantially the same requirements as Embodiment 1. A further detailed description of how they are arranged with be set forth below.

In one embodiment, the coil 20 may be constructed by joining together one O-shaped element 13, two C-shaped elements 12 and two Ω-shaped elements 11 sequentially in the following order: the O-shaped element 13, one of the Ω-shaped elements 11, one of the C-shaped elements 12, the other one of the C-shaped elements 12 and the other one of the Ω-shaped elements 11. One of the C-shaped elements 12 may be arranged at a top side of the pentahedron, and the other C-shaped element 12 may be arranged at a rear side of the pentahedron, i.e., the furthermost side as viewed in Fig. 4. In order to achieve higher deflectability of the S-shaped structure, the top side preferably forms an angle of 90° with the lateral side. In this arrangement, on each of three axes of the pentahedron, there is arranged one Ω-shaped element 11 or O-shaped element 13. This can ensure good stability of the coil 20, in addition to good overall compliance thereof assured by the three-dimensional S-shaped structure. Each lateral side of the pentahedron is preferably perpendicular to both bottom and top sides of the pentahedron, while angles between the lateral sides are not limited to any particular value. Two adjacent ones of the lateral sides may form an angle of 90°.

In alternative embodiments, the coil 20 may be constructed by joining together one O-shaped element 13, two C-shaped elements 12 and two Ω-shaped elements 11 sequentially in the following order: the O-shaped element 13, one of the C-shaped elements 12, the other one of the C-shaped elements 12, one of the Ω-shaped elements 11 and the other one of the Ω-shaped elements 11. In yet alternative embodiments, the coil 20 may be constructed by joining together one O-shaped element 13, three C-shaped elements 12 and one Ω-shaped element 11 sequentially in the following order: the O-shaped element 13, one of the C-shaped elements 12, another one of the C-shaped elements 12, the remaining one of the C-shaped elements 12 and the Ω-shaped element 11. In still alternative embodiments, the coil 20 may be constructed by joining together one O-shaped element 13, three C-shaped elements 12 and one Ω-shaped element 11 sequentially in the following order: the O-shaped element 13, the Ω-shaped element 11, one of the C-shaped elements 12, another one of the C-shaped elements 12 and the remaining one of the C-shaped elements 12.

Similarly, those skilled in the art may make suitable modifications to the foregoing element arrangements in light of the teachings herein to obtain coils 20 with different element arrangements, which are, however, intended to also fall within the scope of the invention. Moreover, in this embodiment, the O-shaped element 13 may be replaced with a Ω-shaped element 11, or may not be the leading element, unless it is arranged at the furthermost or closest end of the embolization device. Moreover, the coil may include more than one O-shaped element 13.

### <Embodiment 3>

A coil 30 according to this embodiment is formed by joining together structural elements, which are of three types and arranged in eight planes. Similarly, the structural elements include O-, Ω- and C-shaped elements 13, 11, 12. There is one O-shaped element 13 and at least two C-shaped elements 12, and the total number of the O-, Ω- and C-shaped elements 13, 11, 12 is eight. The eight structural elements are arranged in the respective eight planes to achieve a coil 30 of an octahedron structure. Herein, the coil 30 generally appears as an octahedron. The octahedron is not limited to a regular octahedron, as long as the structural elements are arranged in respective eight planes of a polyhedron.

In the octahedron, the O-, Ω- and C-shaped elements 13, 11, 12 may also be arranged in many ways, and the arrangement may follow substantially the same requirements as Embodiment 1. A further detailed description of how they are arranged with be set forth below.

In a preferred embodiment, the coil 30 may be constructed by joining together one O-shaped element 13, six C-shaped elements 12 and one Ω-shaped element 11 sequentially in the following order: the O-shaped element 13, one of the C-shaped elements 12, another one of the C-shaped elements 12, a further one of the C-shaped elements 12, a further one of the C-shaped elements 12, a further one of the C-shaped elements 12, the remaining one of the C-shaped elements 12 and the Ω-shaped element 11. Each adjacent pair of the C-shaped elements 12 is arranged at a respective adjacent pair of the planes, and each adjacent pair of the planes where a respective adjacent pair of the C-shaped elements 12 is arranged may form an angle in the range of 100°-120°, with 109°-110° being more preferred. In an alternative embodiment, the coil 30 may be constructed by joining together one O-shaped element 13, five C-shaped elements 12 and two Ω-shaped elements 11 sequentially in the following order: the O-shaped element 13, one of the Ω-shaped elements 11, one of the C-shaped elements 12, another one of the C-shaped elements 12, a further one of the C-shaped elements 12, a further one of the C-shaped elements 12, the remaining one of the C-shaped elements 12 and the other one of the Ω-shaped elements 11, or in the following order: the O-shaped element 13, one of the C-shaped elements 12, another one of the C-shaped elements 12, a further one of the C-shaped elements 12, the other one of the Ω-shaped elements 11, a further one of the C-shaped elements 12, a further one of the C-shaped elements 12 and the remaining one of the C-shaped elements 12.

In this embodiment, the coil 30 is more compliant because it contains more C-shaped elements 12. Compared to the hexahedron or pentahedron, the octahedron has more planes where the additional C-shaped elements 12 can be arranged, which result in an increase in the coil's compliance.

Of course, the present invention is not limited to the above arrangements, and those skilled in the art may modify the above description by making appropriate modification to the details therein so that the modified description is applicable to coils with different arrangements. Similar to Embodiments 1-2, the O-shaped element 13 may be replaced with a Ω-shaped element 11, or may not be the leading element, unless it is arranged at the furthermost or closest end of the embolization device. Moreover, the coil may include more than one O-shaped element 13.

While the pentahedron, hexahedron and octahedron-shaped coils are proposed in Embodiments 1 to 3, the present invention is not limited thereto, because other polyhedron shapes with more planes in which additional C-shaped elements can be arranged to result in a further increase in the coil's compliance are also possible. According to the present invention, a coil must consist of at least four structural elements, which are joined together and arranged in four planes of a polyhedron.

Further, C-shaped elements in a single coil may have either the same or different sizes, for example, in terms of diameter or arc length, and when there are multiple Ω-shaped elements, they may have either the same or different sizes. In one embodiment, the tubular body of the coil may be formed as a primary coil by densely winding a platinum tungsten alloy filament with a diameter ranging from 0,025 mm (0.001 inches) to 0,089 mm (0.0035 inches) onto a metal core rod with a diameter of 0,20 mm (0.008 inches) After that, the primary coil (i.e., the tubular body) may be shaped with the predetermined shape so as to form a coil with polyhedron shape consisting of mutually joined O-, Ω- and S-shaped structural elements.

### <Embodiment 4>

An embolization device according to this embodiment includes the coils 10, 20 or 30 of Embodiment 1-3 for treatment of an aneurysm by thrombus formation in the lumen of the aneurysm. In Embodiment 4, including at least one coil, preferably multiple coils, the multiple coils may be joined together side-by-side and end-to-end in such a manner that in any adjacent pair of the coils, one is swiveled about an axis of the embolization device with respect to the other. With this design, the embolization device has improved stability in various directions, resulting in an increase in overall stability of the embolization device. The embolization device can fill the lumen of the aneurysm in different planes, thus achieving even packing thereof. Further, the swivel ability means increased compliance of the embolization device. The physician may select alternative configurations of the embolization device suitable for the shapes and sizes of particular aneurysms.

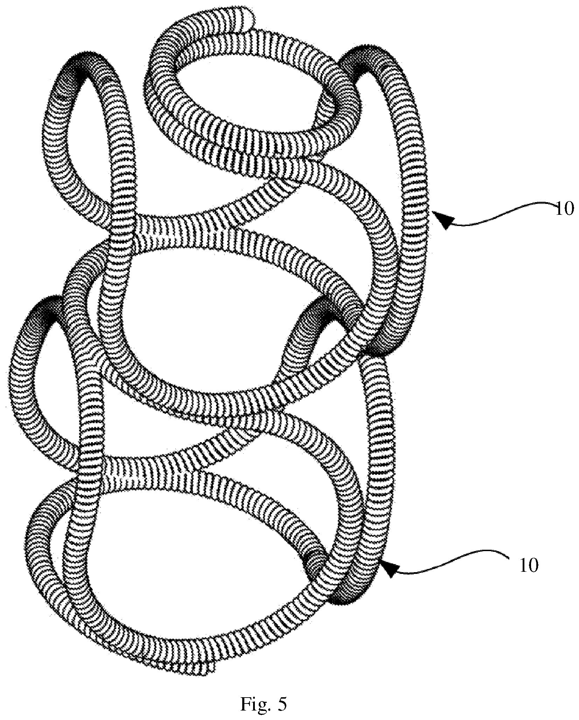

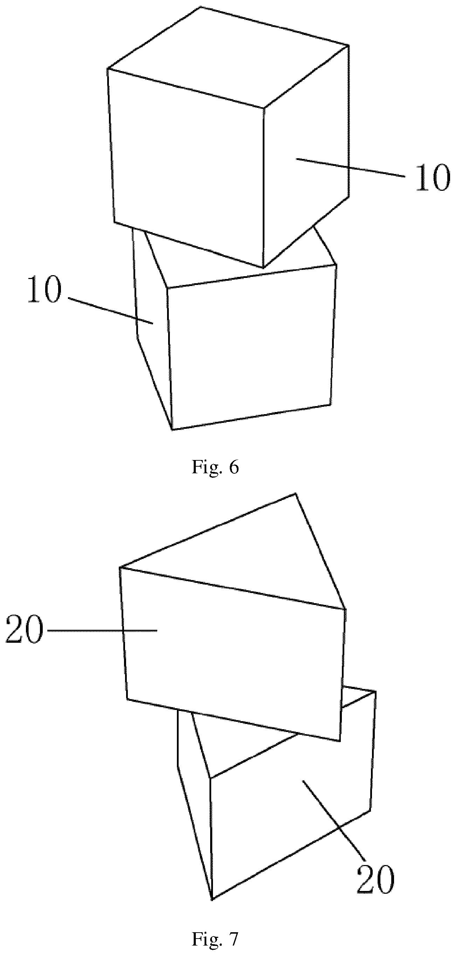

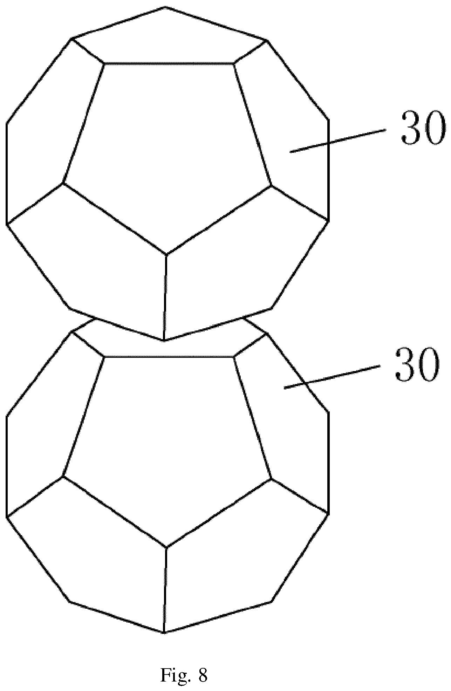

As shown in Fig. 5, the embolization device may be a combination of two or more coils 10, and in any two of the coils 10, one may be swiveled about the axis of the embolization device at an angle of preferably 0°-90°, more preferably 30°, 45°, 60° or 90°. This allows the embolization device to have increased overall stability and enhanced compliance. It should be noted that the two coils 10 may be formed from a single coil tubular body made by coiling a metal, alloy or polymer filament into a spiral (i.e., the primary coil). Fig. 6 is a simplified schematic of two hexahedron-shaped coils 10 which are swiveled with respect to each other, and Fig. 7 is a simplified schematic of two pentahedron-shaped coils 20 which are also swiveled with respect to each other. Apparently, compared with those consisting of single coil, the embolization device constructed from two or more mutually swiveled coils 20 has higher stability and better dense packing performance. Similarly, as shown in Fig. 8, the embolization device may also be made up of a combination of two or more octahedron-shaped coils 30. In Fig. 8, for easier illustration, the octahedron-shaped coils 30 are depicted in a simplified manner.

In this embodiment, the side-by-side arranged coils 10 may be of the same or different sizes and shapes. While the two coils 10 are shown in Fig. 5 to have different structures, the present invention is not limited to this.

In summary, in embolization devices and coils thereof according to embodiments of the present invention, each coil is formed by joining together at least four structural elements arranged in different planes, which include at least two C-shaped elements and at least one O-shaped or Ω-shaped element. The at least two C-shaped elements arranged in two adjacent ones of the planes and sequentially joined together to form an S-shaped structure. The S-shaped structure is highly deflectable and compressive, making the coil easy to change its shape to adapt to different aneurysm shapes. Moreover, the Ω- and O-shaped elements are highly resistant to compression and can provide strong support and ensure sufficient stability of coil, making the coil adaptive to aneurysms of different shapes and sizes. All of these make the coil enables stable, compliant, dense embolization. In addition, each embolization device may include plurality of coils, for example, 2-10 coils, in which any adjacent two can be swiveled with respect to each other about an axis of the embolization device. This allows not only improved packing compliance of the embolization device but also higher overall stability of the packed embolization device, resulting in an even better aneurysm embolization effect.

The embodiments disclosed herein are described in a progressive manner, with the description of each embodiment focusing on its differences from others. Reference can be made between the embodiments for their identical or similar parts.

## Claims

1. An embolization coil (10,20,30) formed by joining together at least four structural elements arranged in different planes, wherein the at least four structural elements include at least two C-shaped elements (12), at least one O-shaped element (13) and at least one Ω-shaped element (11), and wherein the at least two C-shaped elements (12) are arranged in two adjacent planes and are sequentially joined together to form an S-shaped structure, **characterized in that** the at least two C-shaped elements (12) and the at least one Ω-shaped element are open loops each having an opening and wherein the opening of the Ω-shaped element (11) is smaller than the opening of the C-shaped element (12).

2. The embolization coil (10,20,30) of claim 1, wherein the two adjacent planes where the S-shaped structure is arranged form an angle of 60°-120°.

3. The embolization coil (10,20,30) of claim 2, wherein the angle formed by the two adjacent planes where the S-shaped structure is arranged is 90°.

4. The embolization coil (10,20,30) of claim 1, wherein the Ω-shaped element (11) has an arc length greater than or equal to 75% and smaller than 100% of a complete circumference, and wherein the C-shaped (12) element has an arc length greater than or equal to 50% and smaller than 75% of a complete circumference.

5. The embolization coil (10,20,30) of any one of claims 1 to 4, wherein a total number of the structural elements is six, and wherein a number of the C-shaped elements (12) is two.

6. The embolization coil (10,20,30) of claim 5, wherein the six structural elements make up a hexahedron, and wherein one Ω-shaped element (11) is arranged on each of three axes of the hexahedron.

7. The embolization coil (10,20,30) of claim 5, wherein the embolization coil (10,20,30) is formed by sequentially joining O-shaped (13), Ω-shaped (11), C-shaped (12), C-shaped (12), Ω-shaped (11) and Ω-shaped elements (11), which are respectively arranged in different planes.

8. The embolization coil (10,20,30) of any one of claims 1 to 4, wherein a total number of the structural elements is eight, and wherein a number of the C-shaped elements (12) is six.

9. The embolization coil (10,20,30) of claim 8, wherein the embolization coil (10,20,30) is formed by sequentially joining O-shaped (13), C-shaped (12), C-shaped (12), C-shaped (12), C-shaped (12), C-shaped (12), C-shaped (12) and Ω-shaped elements (11), which are respectively arranged in different planes.

10. The embolization coil (10,20,30) of any one of claims 1 to 4, wherein the embolization coil (10,20,30) is formed by winding a tubular body.

11. The embolization coil (10,20,30) of claim 10, wherein the tubular body is made of a metal, alloy or polymer filament and is wound into a spiral.

12. An embolization device comprising at least one embolization coil (10,20,30) as defined in any one of claims 1 to 11.

13. The embolization device of claim 12, wherein the embolization device comprises a plurality of said embolization coils (10,20,30), wherein the plurality of embolization coils (10,20,30) are joined together side-by-side and end-to-end, and wherein in any adjacent two embolization coils (10,20,30), one embolization coil (10,20,30) is swiveled about an axis of the embolization device with respect to the other embolization coil (10,20,30).

14. The embolization device of claim 13, wherein in any adjacent two embolization coils (10,20,30), one embolization coil (10,20,30) is swiveled about the axis of the embolization device with respect to the other embolization coil (10,20,30) by an angle of 0°-90°

15. The embolization device of claim 12, wherein the structural element arranged at a furthermost end of the embolization device is the O-shaped element (13).

## Patentansprüche

1. Embolisationsspirale (10, 20, 30), die durch Zusammenfügen von mindestens vier Strukturelementen geformt ist, die in unterschiedlichen Ebenen angeordnet sind, wobei die mindestens vier Strukturelemente mindestens zwei C-förmige Elemente (12), mindestens ein O-förmiges Element (13) und mindestens ein Ω-förmiges Element (11) einschließen und wobei die mindestens zwei C-förmigen Elemente (12) in zwei benachbarten Ebenen angeordnet sind und aufeinanderfolgend zusammengefügt sind, um eine S-förmige Struktur zu bilden, **dadurch gekennzeichnet, dass** die mindestens zwei C-förmigen Elemente (12) und das mindestens eine Ω-förmige Element offene Schleifen sind, die jeweils eine Öffnung aufweisen, und wobei die Öffnung des Ω-förmigen Elements (11) kleiner ist als die Öffnung des C-förmigen Elements (12).

2. Embolisationsspirale (10, 20, 30) nach Anspruch 1, wobei die zwei benachbarten Ebenen, wo die S-förmige Struktur angeordnet ist, einen Winkel von 60° bis 120° bilden.

3. Embolisationsspirale (10, 20, 30) nach Anspruch 2, wobei der Winkel, der durch die zwei benachbarten Ebenen gebildet wird, wo die S-förmige Struktur angeordnet ist, 90° beträgt.

4. Embolisationsspirale (10, 20, 30) nach Anspruch 1, wobei das Ω-förmige Element (11) eine Bogenlänge, größer als oder gleich 75 % und kleiner als 100 % eines vollständigen Umfangs, aufweist und das C-förmige Element (12) eine Bogenlänge, größer als oder gleich 50 % und kleiner als 75 % eines vollständigen Umfangs, aufweist.

5. Embolisationsspirale (10, 20, 30) nach einem der Ansprüche 1 bis 4, wobei eine Gesamtanzahl der Strukturelemente sechs beträgt und wobei eine Anzahl der C-förmigen Elemente (12) zwei beträgt.

6. Embolisationsspirale (10, 20, 30) nach Anspruch 5, wobei die sechs Strukturelemente ein Hexaeder bilden und wobei ein Ω-förmige Element (11) auf jeder von drei Achsen des Hexaeders angeordnet ist.

7. Embolisationsspirale (10, 20, 30) nach Anspruch 5, wobei die Embolisationsspirale (10, 20, 30) durch aufeinanderfolgendes Zusammenfügen von O-förmigen (13), Ω-förmigen (11), C-förmigen (12), C-förmigen (12), Ω-förmigen (11) und Ω-förmigen Elementen (11) geformt ist, die jeweils in unterschiedlichen Ebenen angeordnet sind.

8. Embolisationsspirale (10, 20, 30) nach einem der Ansprüche 1 bis 4, wobei eine Gesamtanzahl der Strukturelemente acht beträgt und wobei eine Anzahl der C-förmigen Elemente (12) sechs beträgt.

9. Embolisationsspirale (10, 20, 30) nach Anspruch 8, wobei die Embolisationsspirale (10, 20, 30) durch aufeinanderfolgendes Zusammenfügen von O-förmigen (13), C-förmigen (12), C-förmigen (12), C-förmigen (12), C-förmigen (12), C-förmigen (12), C-förmigen (12) und Ω-förmigen Elementen (11) geformt ist, die jeweils in unterschiedlichen Ebenen angeordnet sind.

10. Embolisationsspirale (10, 20, 30) nach einem der Ansprüche 1 bis 4, wobei die Embolisationsspirale (10, 20, 30) durch Wickeln eines röhrenförmigen Körpers geformt ist.

11. Embolisationsspirale (10, 20, 30) nach Anspruch 10, wobei der röhrenförmige Körper aus einem Metall-, Legierungs- oder Polymerfilament hergestellt ist und zu einer Spirale gewickelt ist.

12. Embolisationsvorrichtung, die mindestens eine Embolisationsspirale (10, 20, 30) nach einem der Ansprüche 1 bis 11 umfasst.

13. Embolisationsvorrichtung nach Anspruch 12, wobei die Embolisationsvorrichtung eine Vielzahl der Embolisationsspiralen (10, 20, 30) umfasst, wobei die Vielzahl von Embolisationsspiralen (10, 20, 30) nebeneinander und hintereinander zusammengefügt sind und wobei unter beliebigen zwei benachbarten Embolisationsspiralen (10, 20, 30) die eine Embolisationsspirale (10, 20, 30) um eine Achse der Embolisationsvorrichtung in Bezug auf die andere Embolisationsspirale (10, 20, 30) geschwenkt ist.

14. Embolisationsvorrichtung nach Anspruch 13, wobei unter beliebigen zwei benachbarten Embolisationsspiralen (10, 20, 30) eine Embolisationsspirale (10, 20, 30) um die Achse der Embolisationsvorrichtung in Bezug auf die andere Embolisationsspirale (10, 20, 30) um einen Winkel von 0° bis 90° geschwenkt ist.

15. Embolisationsvorrichtung nach Anspruch 12, wobei das Strukturelement, das an einem entferntesten Ende der Embolisationsvorrichtung angeordnet ist, das O-förmige Element (13) ist.

## Revendications

1. Spire d'embolisation (10, 20, 30) formée en joignant ensemble au moins quatre éléments structurels agencés dans des plans différents, dans laquelle les au moins quatre éléments structurels incluent au moins deux éléments en forme de C (12), au moins un élément en forme de O (13) et au moins un élément en forme de Ω (11), et dans laquelle les au moins deux éléments en forme de C (12) sont agencés dans deux plans adjacents et sont joints ensemble séquentiellement pour former une structure en forme de S, **caractérisée en ce que** les au moins deux éléments en forme de C (12) et l'au moins un élément en forme de S2 sont des boucles ouvertes dont chacune comporte une ouverture et dans laquelle l'ouverture de l'élément en forme de Ω (11) est plus petite que l'ouverture de l'élément en forme de C (12).

2. Spire d'embolisation (10, 20, 30) selon la revendication 1, dans laquelle les deux plans adjacents où la structure en forme de S est agencée forment un angle de 60° à 120°.

3. Spire d'embolisation (10, 20, 30) selon la revendication 2, dans laquelle l'angle formé par les deux plans adjacents où la structure en forme de S est agencée est de 90°.

4. Spire d'embolisation (10, 20, 30) selon la revendication 1, dans laquelle l'élément en forme de Ω (11) présente une longueur d'arc supérieure ou égale à 75 % et inférieure à 100 % d'une circonférence complète, et dans laquelle l'élément en forme de C (12) présente une longueur d'arc supérieure ou égale à 50 % et inférieure à 75 % d'une circonférence complète.

5. Spire d'embolisation (10, 20, 30) selon l'une quelconque des revendications 1 à 4, dans laquelle un nombre total des éléments structurels est de six, et dans laquelle un nombre des éléments en forme de C (12) est de deux.

6. Spire d'embolisation (10, 20, 30) selon la revendication 5, dans laquelle les six éléments structurels constituent un hexaèdre, et dans laquelle un élément en forme de Ω (11) est agencé sur chacun de trois axes de l'hexaèdre.

7. Spire d'embolisation (10, 20, 30) selon la revendication 5, dans laquelle la spire d'embolisation (10, 20, 30) est formée enjoignant séquentiellement des éléments en forme de O (13), en forme de Ω (11), en forme de C (12), en forme de C (12), en forme de Ω (11) et en forme de Ω (11), lesquels sont respectivement agencés dans des plans différents.

8. Spire d'embolisation (10, 20, 30) selon l'une quelconque des revendications 1 à 4, dans laquelle un nombre total des éléments structurels est de huit, et dans laquelle un nombre des éléments en forme de C (12) est de six.

9. Spire d'embolisation (10, 20, 30) selon la revendication 8, dans laquelle la spire d'embolisation (10, 20, 30) est formée en joignant séquentiellement des éléments en forme de O (13), en forme de C (12), en forme de C (12), en forme de C (12), en forme de C (12), en forme de C (12), en forme de C (12) et en forme de Ω (11), lesquels sont respectivement agencés dans des plans différents.

10. Spire d'embolisation (10, 20, 30) selon l'une quelconque des revendications 1 à 4, dans laquelle la spire d'embolisation (10, 20, 30) est formée en enroulant un corps tubulaire.

11. Spire d'embolisation (10, 20, 30) selon la revendication 10, dans laquelle le corps tubulaire est réalisé à partir d'un métal, d'un alliage ou d'un filament en polymère et est enroulé en spirale.

12. Dispositif d'embolisation comprenant au moins une spire d'embolisation (10, 20, 30) telle que définie selon l'une quelconque des revendications 1 à 11.

13. Dispositif d'embolisation selon la revendication 12, dans lequel le dispositif d'embolisation comprend une pluralité desdites spires d'embolisation (10, 20, 30), dans lequel la pluralité de spires d'embolisation (10, 20, 30) sont jointes ensemble côte à côte et bout-à-bout, et dans lequel, parmi deux quelconques spires d'embolisation adjacentes (10, 20, 30), une spire d'embolisation (10, 20, 30) est pivotée autour d'un axe du dispositif d'embolisation par rapport à l'autre spire d'embolisation (10, 20, 30).

14. Dispositif d'embolisation selon la revendication 13, dans lequel, parmi deux quelconques spires d'embolisation adjacentes (10, 20, 30), une spire d'embolisation (10, 20, 30) est pivotée autour de l'axe du dispositif d'embolisation par rapport à l'autre spire d'embolisation (10, 20, 30) sur un angle de 0° à 90°.

15. Dispositif d'embolisation selon la revendication 12, dans lequel l'élément structurel agencé à une extrémité la plus éloignée du dispositif d'embolisation est l'élément en forme de O (13).
